# EUROPEAN PATENT SPECIFICATION

(11) **EP 2 432 408 B1**
(45) Date of publication and mention of the grant of the patent: **30.03.2016**
(21) Application number: 10736809.4
(22) Date of filing: 13.05.2010
(51) Int. Cl.: A61B 17/34

(54) **OBTURATOR FOR TROCAR AND RELATED TROCAR**
OBTURATOR FÜR TROKAR UND ZUGEHÖRIGER TROKAR
OBTURATEUR POUR TROCART ET TROCART ASSOCIÉ

(30) Priority: 19.05.2009 IT RM20090256
(43) Date of publication of application: 28.03.2012
(73) Proprietor: AB Medica Holding S.p.A., 20123 Milano (IT)
(72) Inventor: ORLANDI, Fabio, 20123 Milano (MI) (IT)
(74) Representative: Spina, Alessandro
(86) International application number: PCT/IT2010/000210
(87) International publication number: WO 2010/134111

(56) References cited:
- EP-A1- 0 495 633
- WO-A2-2008/103400
- US-A- 5 772 680
- US-A- 6 017 356
- US-A1- 2002 013 597
- US-A1- 2005 107 816
- US-A1- 2008 300 617

## Description

The present invention relates to an obturator for trocar, and the related trocar, in particular per laparoscopic surgery, that allows in a manner that is reliable, versatile, simple, comfortable for the operator and safe for the patient to introduce within the patient's abdominal cavity surgical instruments, including optical devices, during interventions performed by laparoscopic technique.

In particular, the obturator according to the invention (and the related trocar) is a disposable sterile surgical device with specific low traumaticity dilating polycarbonate tip.

It is known that laparoscopic surgery has been widely spread in recent years thanks to its reduced invasivity.

In order to perform an intervention with laparoscopic technique, an invasive surgical device called trocar, usually disposable sterile, is used that allows to make a small incision on the abdominal walls through which all the surgical instruments, such as scalpels, washing and/or suction cannulae, and optical devices, needed for the intervention, may be introduced within the abdominal cavity.

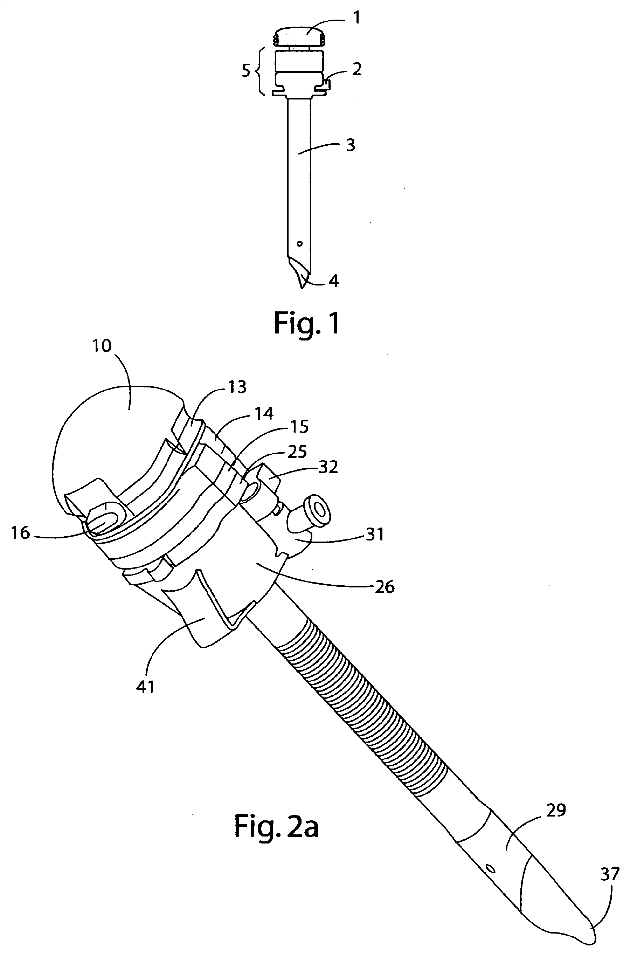

With reference to Figure 1, it may be observed that a trocar comprises a body 5 coupled (usually integrally) to a hollow cylindrical cannula 3 the distal end of which is flared, i.e. cut according to a plane sloped with respect to the plane orthogonal to the longitudinal axis of the cannula 3. A cap 1 is removably coupled to the body 5 and is provided with a cylindrical obturator ending, at its distal end, with a tip 4 capable to perforate tissues that, usually, is whistle shaped. When the cap 1 is coupled to the body 5, the obturator is inserted within the cannula 3 from which only the tip 4 protrudes, so that, when the surgeon handles the cap 1-body 5 assembly and presses the tip 4 against the abdominal walls, the tip cuts the tissues of the same walls introducing the distal portion of the cannula 3 in the abdominal cavity. At the end of the incision, the cap 1 and the obturator are removed for allowing the surgical instruments to be introduced, through the cannula 3 operating as passage channel, in the abdominal cavity. The body 5 is provided with an outer valve 2 for connection of external apparatuses for insufflation (e.g. for gas introduction) and irrigation, still through the cannula 3, with the abdominal cavity. The body 5 internally houses further valves (not shown in Figure 1) ensuring seal of the cannula 3, even when surgical instruments are present.

In order to facilitate the incision, in the prior art some trocars are known wherein the tip 4 of the obturator, that may comprise one or more cutting blades or not (trocars without blades are also called bladeless), is helicoidally shaped.

Document WO 2006/061514 describes a cutting trocar provided with a tip having helicoidal cutting edges.

Document US 5662673 describes a trocar having a tip having a helicoidal cutting blade.

Document WO 03/026512 describes a bladeless obturator for trocar, having a helicoidally twisted tip.

However, all prior art trocars suffer from some drawbacks, mainly due to the difficulty of penetrating tissues maintaining a seal between the cannula outer walls and the abdominal walls.

First of all, trocars having an obturator provided with cutting blades must be carefully handled by surgeons, in order to avoid to cause lesions to internal organs or vessels.

Moreover, bladeless trocars usually entail the need for making, by the surgeon, a preliminary incision of tissues for allowing the obturator and the cannula to be introduced within the abdominal cavity.

Also, both obturators provided with cutting blades and bladeless obturators damage tissues into which they penetrate, deteriorating seal properties of interstices between cannula and the same tissues and making post-operative healing of incisions more difficult.

Furthermore, the difficulty of penetrating tissues is augmented by the poor controllability of the head of trocars, i.e. by the poor ergonomics of the cap-body assembly.

In this context, the solution proposed according to the present invention is introduced, allowing to overcome the aforementioned problems.

It is therefore an object of the present invention to allow in a manner that is reliable, versatile, simple, comfortable for the operator and safe for the patient to introduce within the patient's abdominal cavity surgical instruments, such as scalpels, washing and/or suction cannulae, and optical devices, during interventions performed by laparoscopic technique.

It is specific subject matter of this invention a bladeless obturator for trocar, ending at a distal end with a tip having a base and a vertex, characterised in that the tip has a helicoidal shape by following a helicoidal rotation from the base to the vertex ranging from 30° to 60°.

Always according to the invention, the vertex of the tip is misaligned with respect to a longitudinal axis of the obturator, preferably in such a way that the vertex of the tip is spaced from a longitudinal axis of the obturator by a distance ranging from 20% to 75%, more preferably from 30% to 65%, still more preferably from 40% to 55%, even more preferably from 45% to 50% of a radius of a circle circumscribed to the base of the tip.

Still according to the invention, the helicoidal rotation followed by the tip from the base to the vertex may range from 35° to 55°, preferably from 40° to 50°, more preferably from 44° to 47°.

Furthermore according to the invention, a length of the tip from the base to the vertex may be included within the range from 24,5 to 32,5 mm, preferably from 26 to 31 mm, more preferably from 27 to 30 mm, still more preferably from 28 to 29 mm.

Always according to the invention, a distal cylindrical portion and/or a proximal cylindrical portion may have diameter larger than that of a central cylindrical portion.

It is always specific subject matter of this invention a bladeless trocar comprising a body, coupled to a hollow cylindrical cannula in which an obturator is insertable, and a top cap, capable to be removably coupled to the body, characterised in that the obturator is an obturator as previously described.

Always according to the invention, the top cap may be capable to be removably coupled to the body through an elastic U-shaped connection having two side arms interacting with a U-shaped spring that is housed internally to the body, the two side arms of the elastic connection being provided with respective buttons and ending with respective ridges projecting outwards from the "U", the end ridges of the two side arms of the elastic connection being insertable in corresponding through holes of the body, whereby, when in rest position, the spring keeps the two side arms of the elastic connection spaced apart such that the end ridges are locked by corresponding edges of the through holes, whereas when the side buttons are pressed so as to exceed a strength of the spring the end ridges disengage from said edges of the holes thus releasing the connection and the top cap from the body.

Still according to the invention, the body may comprise a duckbill valve contained in a container having an annular cover, an annular element being rotatably, preferably removably, coupled to the cover through rotatably coupling mechanical means, the annular element being provided with actuator means, the annular element and the cover being capable to assume a configuration in which said actuator means opens the duckbill valve.

Furthermore according to the invention, the body may comprise a self-centring seal device housed within a container, the seal device comprising, in order:
- a proximal annular bracket,
- a proximal membrane having a plurality of rigid elastic elements partially overlapping each other to form a conical surface,
- an intermediate membrane, centrally provided with a through hole,
- an intermediate annular bracket,
- a distal membrane having a plurality of soft elastic elements partially overlapping each other to form a conical surface, and
- a distal annular bracket,
wherein the rigid elastic elements of the proximal membrane insert in the central hole of the intermediate membrane and get in contact with the soft elastic elements of the distal membrane, the intermediate membrane being preferably radially movable within the container, whereby the device is also movable within the container.

Always according to the invention, the proximal annular bracket may be provided on the distal surface with a plurality of pins capable to insert in a corresponding plurality of holes with which the rigid elastic elements of the proximal membrane are provided, in a corresponding plurality of holes of the intermediate membrane, and in a corresponding plurality of holes of the intermediate bracket, and the intermediate annular bracket is provided on the distal surface with a plurality of pins capable to insert in a corresponding plurality of holes of the soft elastic elements of the distal membrane and in a corresponding plurality of holes of the distal bracket.

Still according to the invention, the annular element rotatably coupled to the cover may be part of the container housing the self-centring seal device.

The bladeless obturator, preferably in polycarbonate, according to the invention, has a dilating tip thanks to the specific shape of the same tip. In particular, the tip has a specific helicoidal shape that confers to the tip appropriate mechanical characteristics allowing it to easily penetrate within the abdominal cavity with no need for a preliminary incision made by the surgeon.

This allows to minimise the trauma for the tissues during the insertion phase.

Moreover, the absence of blades on the tip eliminates or at least minimises the risk of lesions to internal organs or vessels.

Furthermore, the use and precision in insertion of the trocar provided with the obturator according to the invention are greatly facilitated by the shape, in particular, of the top cap-body assembly, that makes the handle of the trocar extremely handy and ergonomic, allowing the surgeon to have more control of the same. In this regard, the handle allows an optimal grip for both left-handed people and right-handed people and it allows the use in several positions so as to facilitate a good operability keeping a high precision and safety.

The present invention will be now described, by way of illustration and not by way of limitation, according to its preferred embodiments, by particularly referring to the Figures of the enclosed drawings, in which:
Figure 1 shows a schematic right side view of a trocar of the prior art;
Figure 2 shows a perspective view (Fig. 2a), a front view (Fig. 2b), a left side view (Fig. 2c), a top plan view (Fig. 2d), and a rear view (Fig. 2e) of a preferred embodiment of the trocar provided with the obturator according to the invention;
Figure 3 shows a perspective exploded view of the trocar of Figure 2;
Figure 4 shows a perspective view of the elastic connection of the trocar of Figure 2;
Figure 5 shows a perspective view from above of a first component of the body of the trocar of Figure 2;
Figure 6 shows a perspective view from below of a second component of the body of the trocar of Figure 2;
Figure 7 shows a perspective view from below of a third component of the body of the trocar of Figure 2;
Figure 8 shows a perspective view from below of the top cap of the trocar of Figure 2;
Figure 9 shows a perspective view from below of a fourth component of the body of the trocar of Figure 2;
Figure 10 shows a perspective view from above of a fifth component of the body of the trocar of Figure 2;
Figure 11 shows a perspective view from above of the self-centring seal device of the trocar of Figure 2;
Figure 12 shows a perspective view from below (Fig. 12a), a bottom plan view (Fig. 12b), a section view along the cutting plane AA of Figure 12b (Fig. 12c), and a perspective exploded view of the self-centring seal device of Figure 11;
Figure 13 shows a perspective view of a first component (Fig. 13a), of a second component (Fig. 13b), of a third component (Fig. 13c), of a fourth component (Fig. 13d), of a fifth component (Fig. 13e), and of a sixth component (Fig. 13f) of the self-centring seal device of Figure 11;
Figure 14 shows a top plan view (Fig. 14a), a section view along the cutting plane AA of Figure 14a (Fig. 14b), a section view along the cutting plane BB of Figure 14a (Fig. 14c), and a section view along the cutting plane CC of Figure 14a (Fig. 14d) of the trocar of Figure 2;

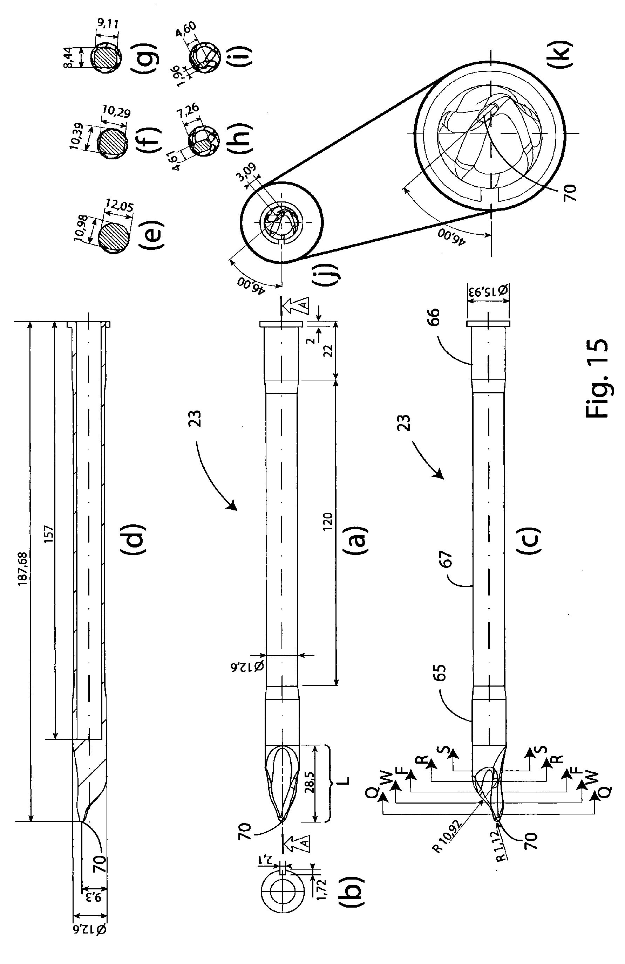

Figure 15 shows a front view (Fig. 15a), a top plan view (Fig. 15b), a left side view (Fig. 15c), a section view along the cutting plane AA of Figure 15a (Fig. 15d), a section view along the cutting plane SS of Figure 15c (Fig. 15e), a section view along the cutting plane RR of Figure 15c (Fig. 15f), a section view along the cutting plane FF of Figure 15c (Fig. 15g), a section view along the cutting plane WW of Figure 15c (Fig. 15h), a section view along the cutting plane QQ of Figure 15c (Fig. 15i), a bottom plan view (Fig. 15j), and an enlarged bottom plan view (Fig. 15k) of the preferred embodiment of the obturator according to the invention;
Figure 16 shows a top plan view (Fig. 16a), a rear view (Fig. 16b), a bottom plan view (Fig. 16c), a section view along the cutting plane FF of Figure 16a (Fig. 16d), a section view along the cutting plane BB of Figure 16a (Fig. 16e), a section view along the cutting plane DD of Figure 16a (Fig. 16f), a section view along the cutting plane HH of Figure 16a (Fig. 16g), a section view along the cutting plane AA of Figure 16a (Fig. 16h), and a section view along the cutting plane GG of Figure 16a (Fig. 16i) of the top cap of the trocar of Figure 2;
Figure 17 shows a top plan view (Fig. 17a), a rear view (Fig. 17b) and a section view along the cutting plane AA of Figure 17a of the third component of Figure 7;
Figure 18 shows a perspective view from above (Fig. 18a), a top plan view (Fig. 18b), a left side view (Fig. 18c), a right side view (Fig. 18d), and a rear view (Fig. 18e) of the fourth component of Figure 9; and
Figure 19 shows five perspective views of a portion of the trocar of Figure 2 in several configurations.

In the Figures, identical reference numbers are used for alike elements.

In particular, dimensions shown in the Figures are by way of example and have not to be intended as limiting the scope of protection of the present invention present invention, unless expressly indicated to the contrary.

In the following, explicit reference will be made to a preferred embodiment of the trocar provided with the obturator according to the invention comprising a top cap having a specific shape. However, it is to be understood that the trocar provided with the obturator according to the invention may comprise any other top cap, having a shape different from that described in the Figures (e.g. with cylindrical symmetry), still remaining within the scope of protection of the present invention.

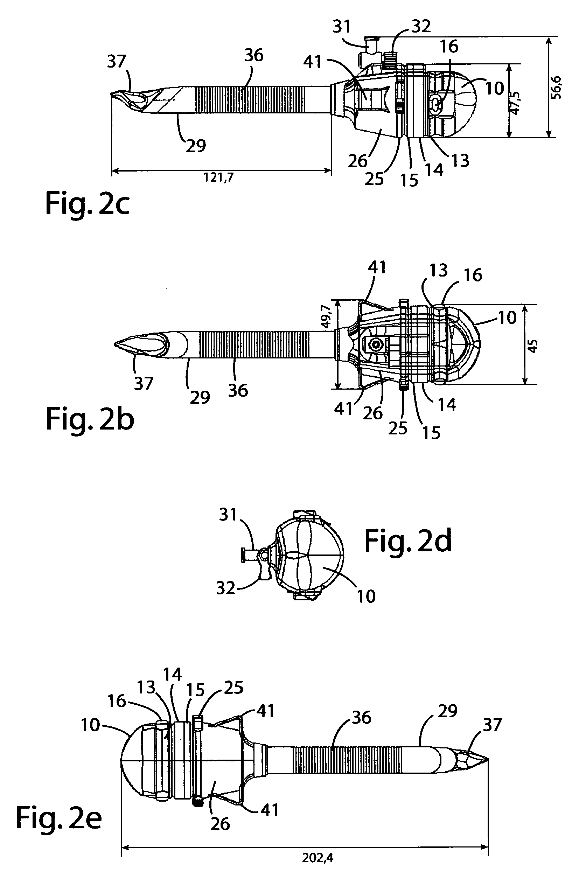

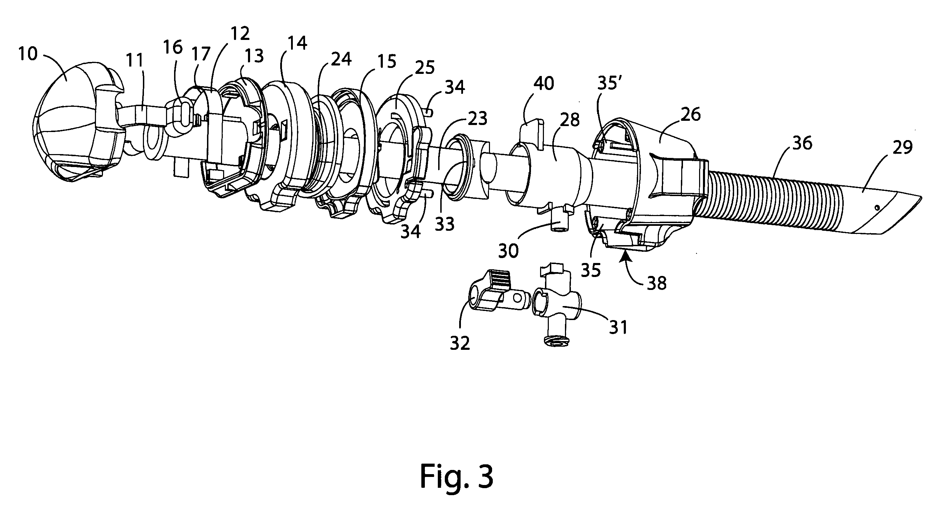

With reference to Figures 2 and 3, it may be observed that the preferred embodiment of the trocar provided with the obturator according to the invention comprises a top cap 10 that may be coupled to a lower cap 13, the latter being centrally provided with a through hole, and to a first annular element 14 through an elastic connection 11 interacting with a U-shaped spring 12, as it will be described in detail in the following.

The first annular element 14 may be in turn coupled to a second annular element 14, so that the first and second annular elements 14 and 15 operate as container of a seal device 24.

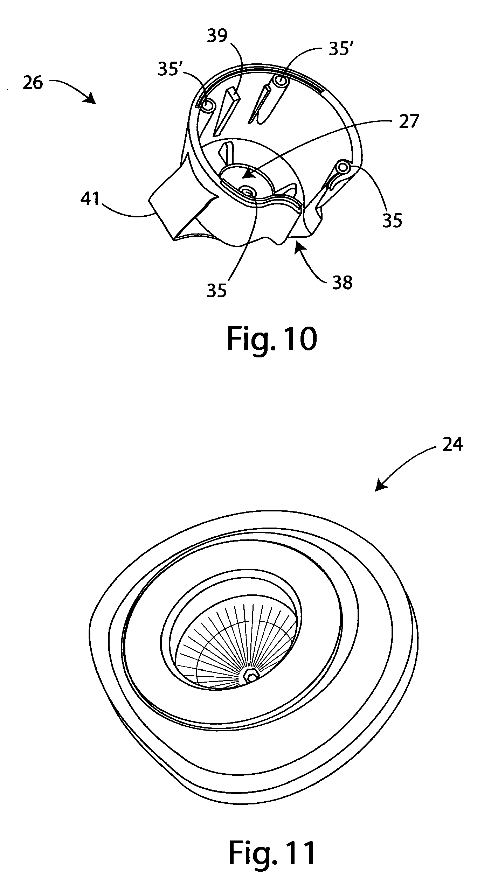

The trocar further comprises an annular cover 25 that is capable to close a container 26, provided at its bottom with a through hole (shown in Figure 10 and there indicated with the reference number 27), that houses the proximal end 28 of a hollow cylindrical cannula 29 (the distal end of which is flared, preferably by 45°). The proximal end 28 of the cannula 29 is provided with a front inlet/outlet duct 30 provided with a tap 31 controlled by a corresponding valve 32, for allowing the inside of the cannula 29 to connect, e.g., with external apparatuses of insufflation (e.g. for introducing gases) and of washing. In this regard, a seal duckbill valve 33 is house in the proximal end 28 of the cannula 29. The outer wall of the cannula 29 is centrally provided with a helicoidal thread 36. In particular, the assembly of components from the bottom cap 13 to the container 26 form the body of the trocar, indicated in Figure 1 with the reference number 5.

The obturator 23, ending with a distal tip 37 having a specific helicoidal shape that will be illustrated below, is capable to insert into the cannula 29.

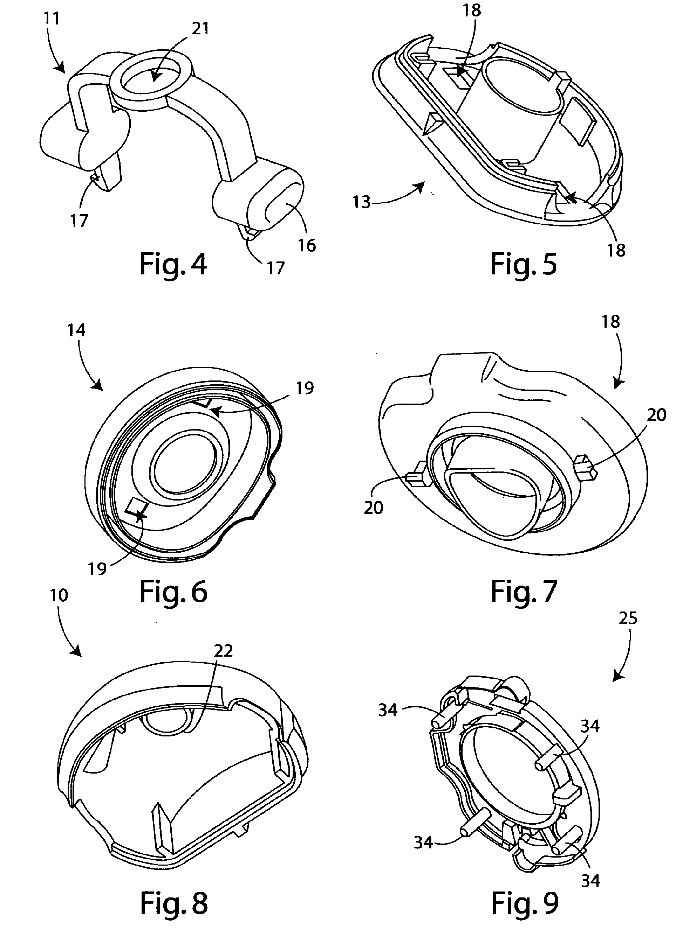

In particular, also with reference to Figure 4, it may be observed that the elastic connection 11 is U-shaped and has the two side arms which are provided, in proximity to the ends, with respective buttons 16 and which end with respective ridges 17 projecting outwards from the U. Making further reference to Figures 5 and 6, when the top cap 10 is assembled (as shown in Figure 2) with the lower cap 13 and the first annular element 14, the end ridges 17 of the side arms of the elastic connection 11 are inserted in corresponding through holes 18 of the lower cap 13 and 19 of the first annular element 14; in this assembled configuration, when in rest position, the spring 12 keeps the side arms of the elastic connection 11 spaced apart (interacting with the surfaces of these facing the inside of the U), so that the end ridges 17 are locked in assembly position by the edges of the holes 19 of the first annular element 14. The top of the intermediate arm connected between the side arms of the elastic connection 11 is provided with a circular through hole 21 into which, in the assembled configuration, a corresponding circular projection 22 of the top of the inner surface of the top cap 10 (shown in Figure 8) is inserted, which constitutes the seat for fixing the trocar obturator 23. Starting from the assembled configuration of Figure 2, by pressing the two side buttons 16 so as to exceed the strength of the spring 12, it is possible to make the ridges 17 of the connection 11 disengage from the edges of the holes 19 of the first annular element 14 and of the holes 18 of the lower cap 13, making the side arms of the connection 11 slide and thus releasing the connection 11 and the top cap 10 from the trocar body, allowing the obturator 23 to be extracted.

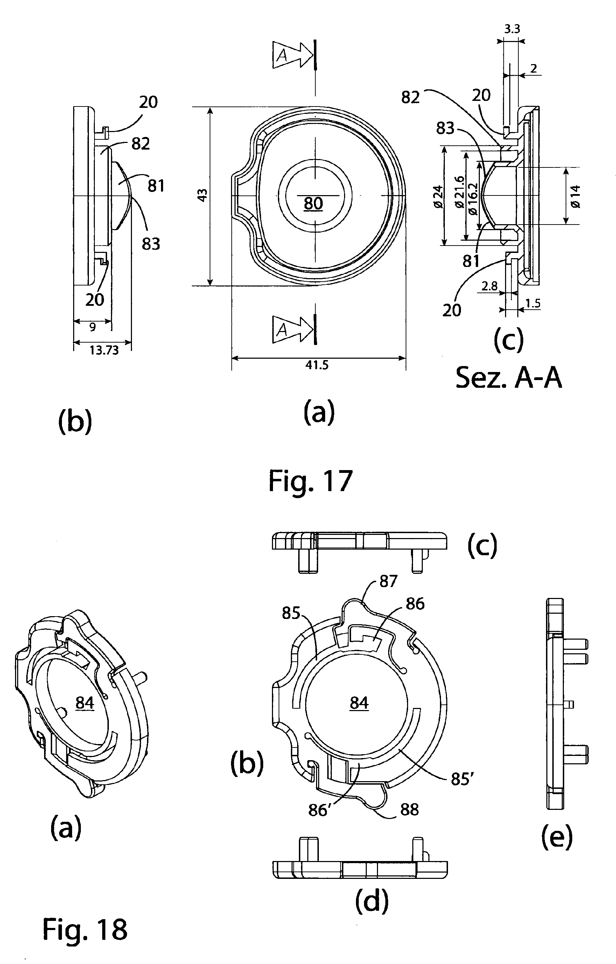

With reference to Figures 7 and 17, it may be observed that the second annular element 15 is provided, on the lower surface, with two ridges 20 and 20' arranged in diametrically opposed positions with respect to the central hole 80, around which, at radially increasing distance, a mechanical actuator 81 and a cylindrical relief 82 (less projecting than the actuator 81) are present. In particular, the mechanical actuator 81 is shaped according to a cylindrical surface sector the distal end of which is curvilinear so as to define two diametrically opposed curvilinear projections 83 (which are located, in the preferred embodiment of the trocar shown in the Figures, along a diameter orthogonal to that along which the two ridges 20 and 20' are located).

With reference to Figures 9 and 10, it may be observed that the annular cover 2 5 is provided at its bottom with four pins 3 4 and th e container 26 is provided with four corresponding seats (the two front ones are indicated with 35 and the two rear ones with 35'): through the insertion of the pins 34 into the seats 35 and 35', the annular cover 25 is capable to be integrally coupled to the container 26. The container 26 is provided at its bottom with a through hole 27 (through which the cannula 29 may slide). The outer wall of the container 26 comprises a front notch 38, wherein the tap 31 and the respective valve 32 may be housed, thus determining the radial position of the cannula 29 within the container 26. Moreover, the inner wall of the con tainer 26 comprises a rear rib 3 9 interacting with a rear tongue 40 of the proximal end 28 of the cannula 29, for determining, along with the cover 25, its longitudinal position within the container 26; in particular, the rear tongue 40 may further interact with the pair of rear seats 35' of the container 26 which (in combination with or alternatively to the housing of the duct 30-tap 31 assembly in the front notch 38) determine its radial position within the same container 26. As resulting also from Figure 2, the container 26 is provided at the distal end with two side projections 41 which allows the trocar to be better handled, as it will be described below.

With reference to Figure 18, it may be observed that the annular cover 25 is further provided, around the central hole 84, with two diametrically opposed guide grooves 85 and 85', shaped as a sector of annulus, each ending at an end with a constraint shaped area, respectively 86 and 86'. In correspondence with the constraint areas 86 and 86', the annular cover 25 comprises two side levers, respectively 87 and 88, which, when operated, allow to modify the shape of the respective areas 86 and 86', allowing the ridges 20 and 20' of the second annular element 25 to slide in the guide grooves 85 and 85' or to disengage the second annular element 25 from the cover 25, as it will be described in detail below.

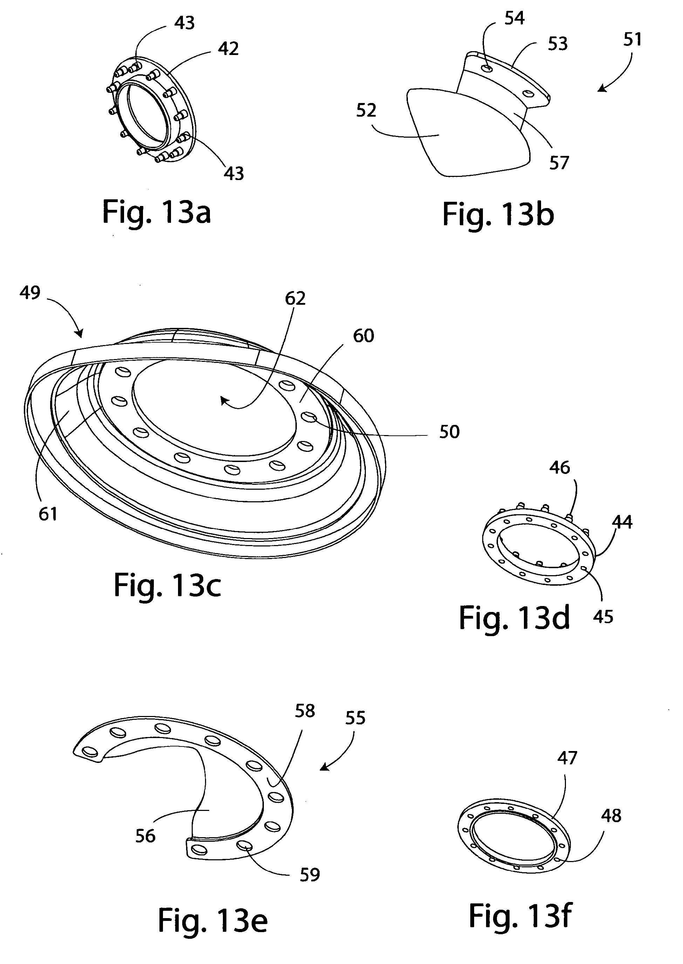

Figures 11-13 show the seal device 24 housed in the first and second annular elements 14 and 15. In particular, the seal device 24, shaped in accordance with the annular elements 14 and 15 (i.e. with circular plan slightly squashed along a frontally placed circle arc, preferably ranging from 90° to 120°) is a self-centring multi-membrane device, comprising:
- a proximal annular bracket 42, provided on the distal surface with a plurality of (preferably 12) pins 43,
- an intermediate annular bracket 44 provided with a plurality of (preferably 12) through holes 45 and, on the distal surface, with a plurality of (preferably 12) pins 46 (obviously misaligned with respect to the holes 45),
- a distal annular bracket 47 provided with a plurality of (preferably 12) through holes 48,
- an intermediate membrane 49 (with plan shaped in accordance with the annular elements 14 and 15), centrally provided with a through hole 62 delimited by an annulus 60 provided with a plurality of (preferably 12) through holes 50, from which membrane a projecting edge 61 (oriented towards the distal end of the trocar) extends,
- a proximal membrane with six rigid elastic elements 51 partially overlapping each other for forming a conical surface, wherein each element 51 comprises a sector 52 of conical surface connected, through a cylindrical sector 57, to a sector 53 of annulus, wherein the conical sector 52 preferably has an angular extent larger than 1/6 of round angle (i.e. 60°) and, preferably, smaller than 3/6 of round angle (i.e. 180°), whereas the circular sector 53 has an angular extent not larger, preferably smaller, than 1/6 of round angle, the circular sector 53 being provided with one or more through holes 54, preferably equal to 1/6 of the number of the plurality of pins 43 of the proximal annular bracket 42 (in the preferred embodiment shown in the Figures such number is equal to two),
- a distal membrane with six soft elastic elements 55 partially overlapping each other for forming a conical surface, wherein each element 55 comprises a sector 56 of conical surface connected to a sector 58 of annulus, wherein the conical sector 56 and the circular sector 58 have an angular extent preferably larger than 1/6 of round angle (i.e. 60°), more preferably larger than 1/2 of round angle (i.e. 180°), and more preferably smaller than 5/6 of round angle (i.e. 300°), the circular sector 58 being provided with one or more through holes 59 (equal to the number of pins 46 of the intermediate bracket 44 included in the angular extent of the circular sector 58; in the preferred embodiment shown in the Figures such number is equal to nine).

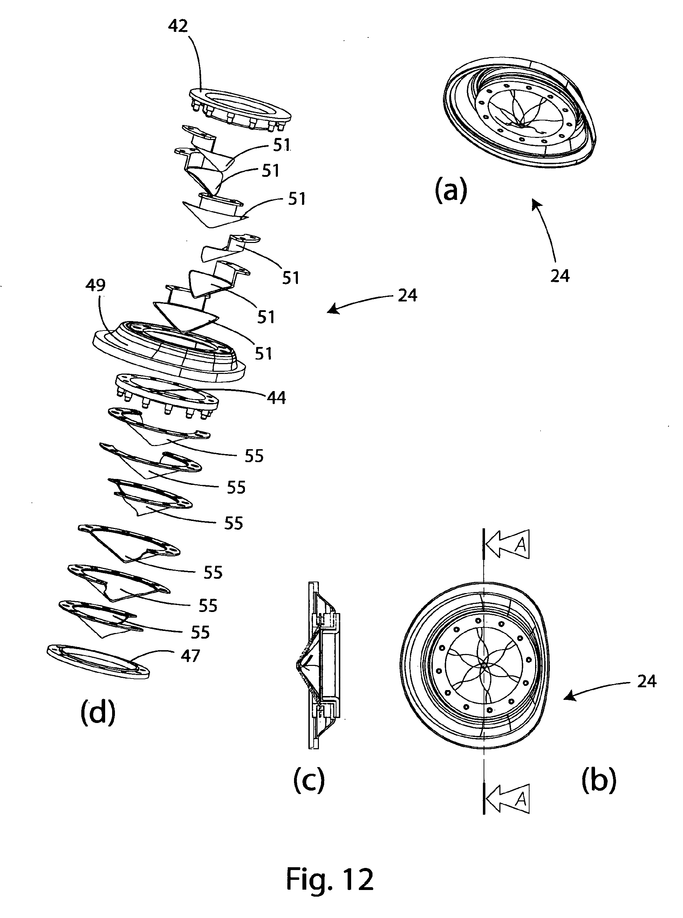

As better shown in Figure 12, the proximal membrane is located between the proximal bracket 42 and the intermediate membrane 49, the intermediate bracket 44 is housed in the intermediate membrane 49 in contact with the distal surface of the annulus 60 and internally to the projecting edge 61, and the distal membrane is located between the intermediate bracket 44 and the distal bracket 47, so that the pins 43 of the proximal bracket 42 are inserted into the holes 54 of the elements 51 of the proximal membrane, into the holes 50 of the intermediate membrane 49, and into the holes 45 of the intermediate bracket 44, and that the pins 46 of the intermediate bracket 44 are inserted into the holes 59 of the elements 55 of the distal membrane and into the holes 48 of the distal bracket 47. In particular, when the device 24 is assembled, the elements 51 of the proximal membrane are inserted into the central hole 62 of the intermediate membrane 49 contacting the elements 55 of the distal membrane. The intermediate membrane 49 is radially movable within the housing formed by the annular elements 14 and 15, allowing the whole device 24 when assembled to have an identical mobility. This allows the insertion of surgical instruments within the abdominal cavity of the patient through the trocar with several angles with respect to the longitudinal axis of the cannula 29.

In particular, when the obturator 13 is inserted into the cannula 29 or at least one surgical instrument is inserted within the patient's abdominal cavity through the trocar, it is the device 24 that ensures the sealing of the abdominal cavity (avoiding leakage of biological fluids, usually maintaining the pressure difference with the exterior); instead, when the obturator 13 is not inserted into the cannula 29 nor any other surgical instrument is inserted within the abdominal cavity, it is the duckbill valve 33 that ensures the sealing of the abdominal cavity.

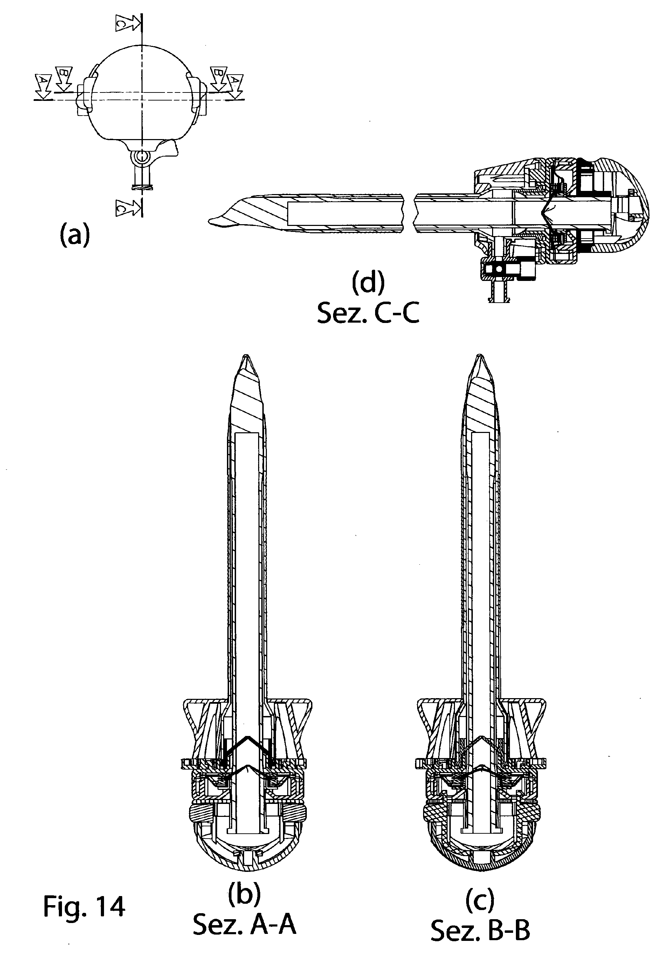

Figure 14 shows some sections of the trocar in the assembled configuration of Figure 2. With regard to the materials with which the trocar components are made, the top and lower caps 10 and 13, the connection 11, the first and second annular elements 14 and 15, the obturator 23, the cover 25, the container 26, the cannula 29, and the tap 31 are preferably made of polycarbonate; the spring 12 is preferably made of spring steel (i.e. still with high rate of carbon, preferably ranging from 0,80% to 0,90 %, hence particularly hard); the valve 32 of the tap 31 is preferably made of polyethylene; the duckbill valve 33 is preferably made of silicone. With regard to the seal device 24, the proximal, intermediate and distal brackets 42, 44 and 47 are preferably made of polycarbonate; the proximal membrane is preferably made of laprene; the intermediate membrane 49 and the distal membrane are preferably made of a mixture of styrene butadiene rubber (SBR) and nitrile rubber (NBR). Moreover, the trocar is assembled by using adhesives of biocompatible type.

Figure 15 shows the obturator 23 of the preferred embodiment of the trocar according to the invention, wherein it may be observed that the distal and proximal portions 65 and 66 of the obturator 23 preferably have diameter larger than the central cylinder 67. The innovative feature of the trocar according to the invention is that the distal tip 37 has a specific helicoidal shape. First of all, also making reference to Figure 2, it may be observed that the tip 37, extending for a length L, follows a helicoidal rotation from the base to the vertex 70 ranging from 30° to 60°, preferably from 35° to 55°, more preferably from 40° to 50°, still more preferably from 44° to 47° (in the preferred embodiment shown in the Figures it is equal to 46°). The maximum diameter of the obturator 23 (and consequently of the cannula 29), that coincides with that of the distal and proximal portions 65 and 66, may vary (preferably, according to standard sizes of 5, 10 and 12 mm). The length L of the tip 37 is fixed at a value preferably variable within the range from 24,5 to 32,5 mm, more preferably from 26 to 31 mm, still more preferably from 27 to 30 mm, even more preferably from 28 to 29 mm (in the preferred embodiment of the trocar shown in the Figures L is equal to 28,5 mm). Moreover, the vertex 70 of the tip 37 is misaligned with respect to the longitudinal axis of the obturator 23; in particular, the vertex 70 (preferably its geometric centroid) is spaced from the axis of the obturator 23 by a distance preferably ranging from 20% to 75% of the radius of the base of the tip 37 (coinciding with the radius of the distal and proximal portions 65 and 66), more preferably from 30% to 65% of such radius, still more preferably from 40% to 55% of such radius, even more preferably from 45% to 50% of such radius (in the preferred embodiment of the trocar shown in the Figures, the radius of the base of the tip 37 - i.e. of the distal and proximal portions 65 and 66 - is equal to 6,3 mm and the vertex 70 is misaligned with respect to the centre by 3,0 mm). In particular, as shown in Figure 2, the obturator 23 is preferably assembled in the trocar so that the vertex 70 of the tip 37 is frontally misaligned, i.e. towards the tap 37. The specific helicoidal shape of the tip 37 of the obturator 23 has particular mechanical characteristics which allow it to easily penetrate by dilating the tissues of the abdominal walls, instead of lacerating or cutting them.

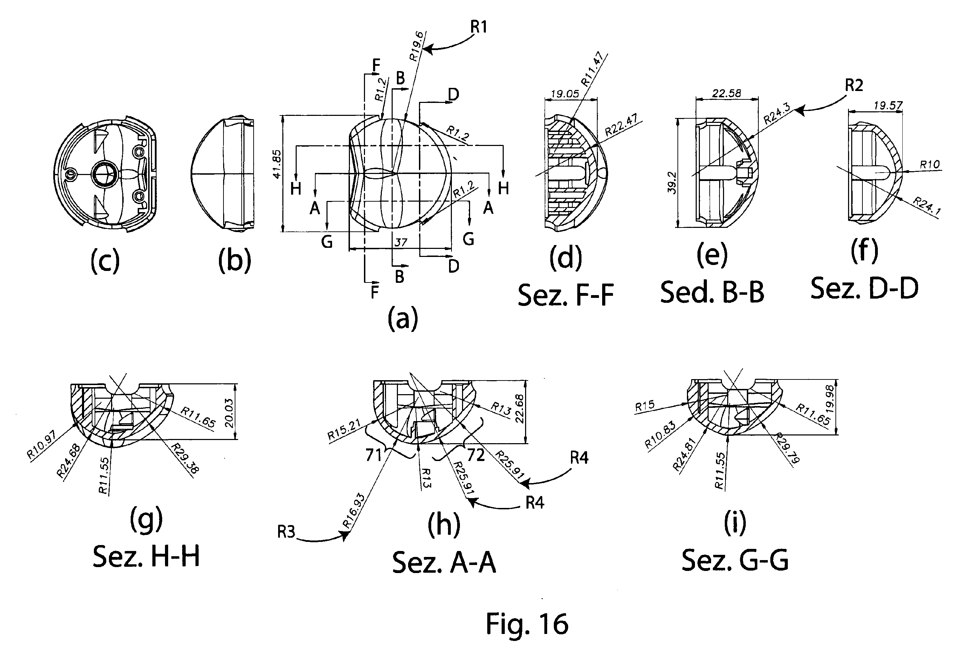

Figure 16 shows in detail the top cap 10, pointing out its shape that renders trocar grip extremely handy and ergonomic. In this regard, it may be observed that the top cap 10 has substantially circular plan, with radius R1, slightly squashed along a frontally located circle arc, preferably ranging from 90° to 120°. The radius R1 is fixed at a value preferably variable within the range from 16,5 to 22,5 mm, more preferably from 17,5 to 21,5 mm, still more preferably from 18,5 to 20,5 mm, even more preferably from 19 to 20 mm (in the preferred embodiment of the trocar shown in the Figures R1 is equal to 19,6 mm).

The section obtained by cutting the top cap 10 with a plane parallel to the housing of the buttons 16 of the connection 11 and passing through the top (shown in Figure 16e) has an upper profile (or proximal profile, belonging to the surface contacting the hand palm of the surgeon handling the trocar) comprising a central portion having a bending radius R2 slightly larger than the plan radius R1. Preferably, the ratio R2/R1 is fixed within the range going from 1,00 to 1,50, more preferably from 1,10 to 1,40, still more preferably from 1,15 to 1,35, even more preferably from 1,20 to 1,30 (in the preferred embodiment shown in the Figures R1 is equal to 19,6 mm and R2 is equal to 24,5 mm).

The section obtained by cutting the top cap 10 with a plane orthogonal to the previous one and passing through the top (shown in Figure 16h) has an upper profile (belonging to the surface contacting the hand palm of the surgeon handling the trocar) comprising a front portion 71 having a bending radius R3 and a rear portion 72 having a bending radius R4. In particular, the front radius R3 is smaller than the rear radius R4; preferably, the front radius R3 is slightly smaller than the plan radius R1, whereas the rear radius R4 is slightly larger than the same radius R1. Preferably, the ratio R3/R1 is fixed within the range going from 0,60 to 1,10, more preferably from 0,70 to 1,00, still more preferably from 0,75 to 0,95, even more preferably from 0,80 to 0,90 (in the preferred embodiment shown in the Figures R1 is equal to 19,6 mm and R3 is equal to 16,93 mm). Preferably, the ratio R4/R1 is fixed within the range going from 1,05 to 1,60, more preferably from 1,15 to 1,50, still more preferably from 1,25 to 1,40, even more preferably from 1,30 to 1,35 (in the preferred embodiment shown in the Figures R1 is equal to 19,6 mm and R4 is equal to 25,91 mm). In the preferred embodiment of the top cap 10 shown in Figure 16, it may be observed that the front and rear portions 71 and 72 of the upper profile are linked by an intermediate portion having bending radius smaller than R3, and that the upper profile further comprises a front end portion, with bending radius slightly smaller than R3, and a rear end portion, with bending radius slightly smaller than R4.

With reference to Figure 2, it may be observed that the whole height of the handle of the trocar according to the invention may be easily adjusted by varying the heights of the component elements, from the top cap 10 down to the container 26, in order to improve handiness and ergonomics of the handle of the trocar.

In general, the device is mostly made by using plastic materials obtained through molding, and the parts are then assembled through glueing, through fixing by pressing or through using screws.

The modes of use of the troc ar according to the invention ar e extremely simple. In fact, still making reference to Figure 2, the surgeon handles the trocar by placing the rear part of the top cap 10 and of the body within the hand palm and using the two distal side projections 41 for having a grip with the fingers (preferably middle and ring fingers); in other words, the trocar body is closed within the fist whereas the cannula 29 thereof comes out from the space between two fingers pressing the two distal side projections 41. During the phase of pushing the trocar, it is advantageous to also impart a slight torsional clockwise movement, facilitating the penetration of the tip 37 of the obturator 23 and of the cannula 29 into the abdominal cavity, the tissues of the walls of which are spaced apart without lacerations thanks to the specific helicoidal shape of the tip 37. This torsional movement further allows the central helicoidal thread 36 of the cannula 29 to adhere to the abdominal walls, facilitating the mechanical coupling of the trocar to the abdomen and eliminating the need for suture stitches or clips for fixing the cannula 29 to the abdominal wall.

Once the introduction of the trocar according to the invention into the abdominal cavity has been completed, the surgeon presses (e.g. with thumb and index finger of a hand) the two side buttons 16 of the connection 11 thus removing the top cap 10 and extracting the obturator 23. During the extraction of the obturator 23, leakage of biological fluids is avoided by the duckbill valve 33 and the seal device 24.

Then, when the obturator 23 has been extracted, the cannula 29 becomes channel for allowing passage of several surgical instruments. It is also possible to introduce medical gases through the tap 31, by connecting it with external insufflation apparatuses preferably through standard luer lock connection.

As said, the use of the trocar according to the invention is facilitated by the shape of the same, that allows a handy and ergonomic handling, wherein the top cap 10 is capable to be stably housed within the hand palm and the two distal side projections 41 offer firm leverage points for the fingers (preferably middle and ring fingers) so as to allow a reliable control by both left- and right-handed surgeons.

Preferably, total length of the trocar, from the top cap 10 to the tip 37 of the obturator 23, does not exceed 180 mm, whereas transverse size of the handle are of the order of 40 mm.

In order to visually differentiate trocar models having different diameter of the obturator 23 and of the corresponding cannula 29 (usually selected out of the standard diameters of 5, 10 and 12 mm), the numbers corresponding to each diameter may be advantageously frontally (i.e. on the side of the tap 31) pad printed on the top cap 10; moreover, also a respective colour may be further pad printed on the top cap 10.

With reference to Figures 7, 9 and 17-19, it may be observed that the second annular element 15 (coupled to the first annular element 14 so as to contain the seal device 24) and the annular cover 25, in correspondence of which the duckbill valve 33 is, are rotatably coupled to each other so that, thanks to the lower shape of the second annular element 15, they may assume a configuration wherein the duckbill valve 33, that in its rest position is closed, is open.

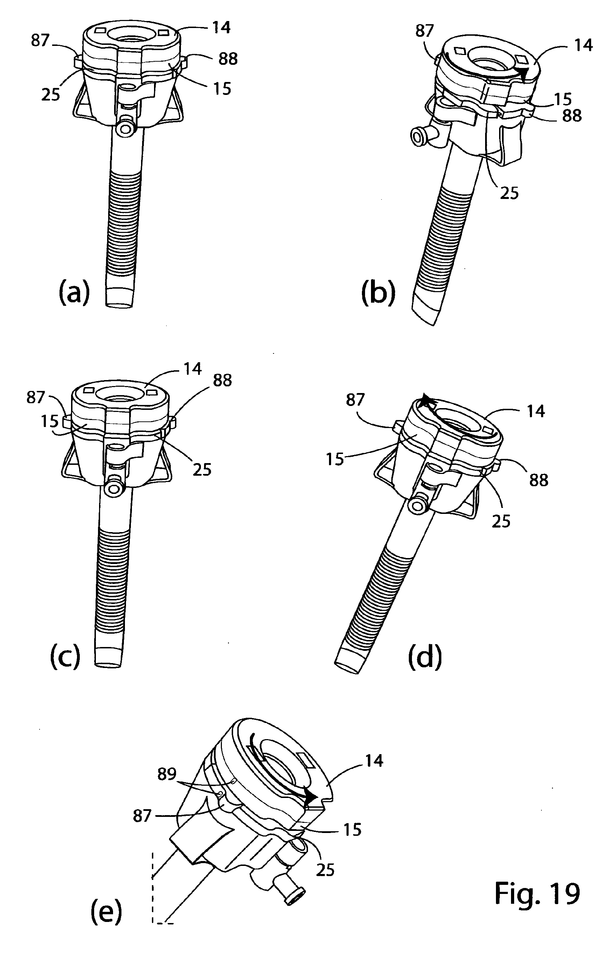

In the assembled configuration of Figure 2, wherein, as shown in Figure 19a, the second annular element 15 (and the first annular element 14) and the annular cover 25 are aligned with each other, the two ridges 20 and 20' of the former are locked in the constraint areas 86 and 86' of the latter.

Starting from this assembled configuration, by keeping the left side opening lever 87 pressed, the shape of the constraint area 86 of the cover 25 is modified allowing the left ridge 20 of the second annular element 15 to disengage from such area 86 and allowing the ridges 20 and 20' of the second annular element 15 to slide within the grooves 85 and 85' of the cover 25, thus rotating the second annular element 15 (and the first annular element 14) anticlockwise with respect to the cover 25 as shown by the arrow in Figure 19b. In this configuration, the two curvilinear projections 83 of the mechanical actuator 81 of the second annular element 15 space the lips of the valve 33 apart thus opening it. From this configuration, it is sufficient to rotate the second annular element 15 (and the first annular element 14) clockwise with respect to the cover 25 for returning to the assembled configuration of Figures 2 and 19a.

In order to remove the first and second annular elements 14 and 15, and the seal device 24 contained therein, it is sufficient, starting from the assembled configuration shown in Figure 19c, keeping the right side closing lever 88 pressed, in such a way that the shape of the constraint area 86' of the cover 25 is modified allowing the right ridge 20' of the second annular element 15 to disengage from such area 86' and allowing the ridges 20 and 20' of the second annular element 15 to slightly rotate clockwise with respect to the cover 25 as shown by the arrow in Figure 19d. In this configuration, the two curvilinear projections 83 of the mechanical actuator 81 of the second annular element 15 space the lips of the valve 33 apart thus opening it. In this configuration, both ridges 20 and 20' are disengaged and free to slide upwards, allowing the second annular element 15 (along with the first annular element 14 and the seal device 24 contained therein) to be uncoupled from the cover 25.

In order to insert the second annular element 15 (along with the first annular element 14 and the seal device 24 contained therein) on the cover 25, it is sufficient to insert the ridges 20 and 20' of the second annular element 15 in the constraint areas 86 and 86' of the cover 25, keeping the second annular element 15 and the cover in the same angular orientation that they have when they are separated (as shown in Figure 19d), and subsequently to rotate the second annular element 15 (along with the first annular element 14 and the seal device 24 contained therein) anticlockwise with respect to the cover 25 as shown by the arrow of Figure 19e, until the assembled configuration (shown in Figure 19a), wherein they are aligned, is reached. Advantageously, two indicators (e.g. two arrows) 89 on the side surface of the second annular element 15 and of the cover 25 may indicate the correct angular orientation of these.

The preferred embodiments have been above described and some modifications of this invention have been suggested, but it should be understood that those skilled in the art can make variations and changes, without so departing from the related scope of protection, as defined by the following claims.

## Claims

1. Bladeless obturator for trocar, ending at a distal end with a tip (37) having a base and a vertex (70), wherein the tip (37) has a helicoidal shape by following a helicoidal rotation from the base to the vertex (70) ranging from 30° to 60°, **characterized in that** the vertex (70) of the tip (37) is misaligned with respect to a longitudinal axis of the obturator (23).

2. Obturator according to claim 1, **characterised in that** the vertex (70) of the tip (37) is spaced from a longitudinal axis of the obturator (23) by a distance ranging from 20% to 75%, more preferably from 30% to 65%, still more preferably from 40% to 55% even more preferably from 45% to 50% of a radius of a circle circumscribed to the base of the tip (37).

3. Obturator according to claim 1 or 2, **characterised in that** the helicoidal rotation followed by the tip (37) from the base to the vertex (70) ranges from 35° to 55°, preferably from 40° to 50°, more preferably from 44° to 47°.

4. Obturator according to any one of the preceding claims, **characterised in that** a length of the tip (37) from the base to the vertex (70) is included within the range from 24,5 to 32,5 mm, preferably from 26 to 31 mm, more preferably from 27 to 30 mm, still more preferably from 28 to 29 mm.

5. Obturator according to any one of the preceding claims, **characterised in that** a distal cylindrical portion (65) and/or a proximal cylindrical portion (66) have diameter larger than that of a central cylindrical portion (67).

6. Bladeless trocar comprising a body (13, 14, 15, 25, 26), coupled to a hollow cylindrical cannula (29) in which an obturator (23) is insertable, and a top cap (10), capable to be removably coupled to the body (13, 14, 15), **characterised by** comprising an obturator according to any one of claims 1 to 5.

7. Trocar according to claim 6, **characterised in that** the top cap (10) is capable to be removably coupled to the body (13, 14, 15, 25, 26) through an elastic U-shaped connection (11) having two side arms interacting with a U-shaped spring (12) that is housed internally to the body (13, 14, 15, 25, 26), the two side arms of the elastic connection (11) being provided with respective buttons (16) and ending with respective ridges (17) projecting outwards from the "U", the end ridges (17) of the two side arms of the elastic connection (11) being insertable in corresponding through holes (18, 19) of the body (13, 14), whereby, when in rest position, the spring (12) keeps the two side arms of the elastic connection (11) spaced apart such that the end ridges (17) are locked by corresponding edges of the through holes (19), whereas when the side buttons (16) are pressed so as to exceed a strength of the spring (12) the end ridges (17) disengage from said edges of the holes (18, 19) thus releasing the connection (11) and the top cap (10) from the body (13, 14, 15, 25, 26).

## Patentansprüche

1. Klingenloser Obturator für einen Trokar, der an einem distalen Ende in einer Spitze (37) endet, die eine Basis und einen Scheitelpunkt (70) aufweist, wobei die Spitze (37) eine schraubenartige Gestalt aufweist, indem sie von der Basis zum Scheitelpunkt (70) einer schraubenförmigen Drehung von 30° bis 60° folgt, **dadurch gekennzeichnet, dass** der Scheitelpunkt (70) der Spitze (37) gegenüber der Längsachse des Obturators verstellt ist.

2. Obturator gemäß Anspruch 1, **dadurch gekennzeichnet, dass** der Scheitelpunkt (70) der Spitze (37) von einer Längsachse des Obturators (23) um einen Abstand entfernt ist, welcher in einem Bereich von 20 % bis 75 % liegt, bevorzugter von 30 % bis 65 %, noch weiter bevorzugt von 40 % bis 55 %, sogar noch weiter bevorzugt von 45 % bis 50 % des Radius eines der Basis der Spitze (37) umbeschriebenen Kreises.

3. Obturator gemäß Anspruch 1 oder 2, **dadurch gekennzeichnet, dass** die schraubenförmige Drehung, welcher die Spitze (37) von der Basis bis zum Scheitelpunkt (70) folgt, von 35° bis 55° reicht, vorzugsweise von 40° bis 50°, besonders bevorzugt von 44° bis 47°.

4. Obturator gemäß einem der vorhergehenden Ansprüche, **dadurch gekennzeichnet, dass** eine Länge der Spitze (37) von der Basis bis zum Scheitelpunkt (70) in einem Bereich von 24,5 bis 32,5 mm enthalten ist, vorzugsweise von 26 bis 31 mm, besonders bevorzugt von 27 bis 30 mm, noch weiter bevorzugt von 28 bis 29 mm.

5. Obturator gemäß einem der vorhergehenden Ansprüche, dass ein distaler, zylindrischer Bereich (65) und/oder ein proximaler, zylindrischer Bereich (66) einen Durchmesser aufweist, welcher größer ist als derjenige eines mittigen, zylindrischen Bereichs (67).

6. Klingenloser Trokar, umfassend einen Körper (13, 14, 15, 25, 26), der an eine hohle zylindrische Kanüle (29) gekoppelt ist, in welche ein Obturator (23) einsetzbar ist, sowie eine obere Kappe (10), welche auf lösbare Weise mit dem Körper (13, 14, 15) verbindbar ist, **dadurch gekennzeichnet, dass** er einen Obturator gemäß einem der Ansprüche 1 bis 5 umfasst.

7. Trokar gemäß Anspruch 6, **dadurch gekennzeichnet, dass** die obere Kappe (10) auf lösbare Weise mit dem Körper (13, 14, 15) verbindbar ist über eine elastische, U-förmige Verbindung (11) mit zwei Seitenarmen, welche mit einer U-förmigen Feder (12) interagieren, die anfänglich in dem Körper (13, 14, 15, 25, 26) untergebracht ist, wobei die beiden Seitenarme der elastischen Verbindung (11) mit je einem Knopf (16) versehen sind und mit je einem von dem "U" nach außen vorstehenden Grat (17) enden, wobei die Endgrate (17) der beiden Seitenarme der elastischen Verbindung (11) in entsprechende Durchgangslöcher (18, 19) des Körpers (13, 14) einsetzbar sind, wobei in der Ruheposition die Feder (12) die beiden Seitenarme der elastischen Verbindung (11) auf Abstand hält, derart, dass die Endgrate (17) durch entsprechende Kanten der Durchgangslöcher (19) verriegelt sind, während die Endgrate (17) von den besagten Kanten der Löcher (18, 19) ausgerückt werden, indem die seitlichen Knöpfe (16) derart zusammengepresst werden, dass eine Federkraft (12) überschritten wird, wodurch die Verbindung (11) und die obere Kappe (10) von dem Körper (13, 14, 15, 25, 26) gelöst wird.

## Revendications

1. Obturateur sans lame pour trocart, se terminant en une extrémité distale avec une pointe (37) ayant une base et un sommet (70), dans lequel la pointe (37) a une forme hélicoïdale en suivant une rotation hélicoïdale depuis la base jusqu'au sommet (70), de 30° à 60°, **caractérisé en ce que** le sommet (70) de la pointe (37) est décalé par rapport à un axe longitudinal de l'obturateur (23).

2. Obturateur selon la revendication 1, **caractérisé en ce que** le sommet (70) de la pointe (37) est espacé d'un axe longitudinal de l'obturateur (23) d'une distance comprise entre 20% et 75%, de préférence entre 30% à 65%, plus préférablement de 40% à 55%, et encore plus préférablement de 45% à 50% d'un rayon d'un cercle circonscrit à la base de la pointe (37).

3. Obturateur selon la revendication 1 ou 2, **caractérisé en ce que** la rotation hélicoïdale suivie par la pointe (37) depuis la base jusqu'au sommet (70) est comprise entre 35° et 55°, de préférence entre 40° et 50°, plus préférablement entre 44° et 47°.

4. Obturateur selon l'une quelconque des revendications précédentes, **caractérisé en ce qu'**une longueur de la pointe (37) de la base au sommet (70) est incluse dans la gamme de 24,5 à 32,5 mm, de préférence de 26 à 31 mm, plus préférablement de 27 à 30mm, et encore plus préférablement de 28 à 29 mm.

5. Obturateur selon l'une quelconque des revendications précédentes, **caractérisé en ce qu'**une partie cylindrique distale (65) et/ou une partie cylindrique proximale (66) ont un diamètre supérieur à celui d'une partie cylindrique centrale (67).

6. Trocart sans lame comprenant un corps (13, 14, 15, 25, 26) raccordé à une canule cylindrique creuse (29), dans lequel un obturateur (23) peut être inséré, et un capuchon supérieur (10) capable d'être raccordé de manière amovible au corps (13, 14, 15), **caractérisé en ce qu'**il comprend un obturateur selon l'une quelconque des revendications 1 à 5.

7. Trocart selon la revendication 6, **caractérisé en ce que** le capuchon supérieur (10) est apte à être raccordé de manière amovible au corps (13, 14, 15, 25, 26) à travers un raccord en forme de U élastique (11), ayant deux bras latéraux interagissant avec un ressort en forme de U (12), qui est abrité à l'intérieur du corps (13, 14, 15, 25, 26), les deux bras latéraux du raccord élastique (11) étant dotés de boutons respectifs (16) et se terminant par des arêtes respectives (17) se projetant vers l'extérieur depuis le « U », les arêtes d'extrémité (17) des deux bras latéraux du raccord élastique (11) pouvant être insérées dans des orifices passants (18, 19) correspondants du corps (13, 14) moyennant quoi, lorsqu'il est en position de repos, le ressort (12) maintient les deux bras latéraux du raccord élastique (11) espacés, de sorte que les arêtes d'extrémité (17) soient bloquées par les bords correspondants des orifices passants (19), tandis que, lorsque les boutons latéraux (16) sont enfoncés de façon à excéder une force du ressort (12), les arêtes d'extrémité (17) se désengagent desdits bords des trous (18, 19) en libérant ainsi la connexion (11) et le capuchon supérieur (10) du corps (13, 14, 15, 25, 26).
